# EUROPÄISCHE PATENTSCHRIFT

(11) **EP 2 671 305 B1**
(45) Veröffentlichungstag und Bekanntmachung des Hinweises auf die Patenterteilung: **15.06.2016**
(21) Anmeldenummer: 12706451.7
(22) Anmeldetag: 30.01.2012
(51) Int. Cl.: H02K 3/24, H02K 3/44, A61C 1/06, H02K 1/27

(54) **ELEKTROMOTOR**
ELECTRIC MOTOR
MOTEUR ÉLECTRIQUE

(30) Priorität: 31.01.2011 DE 102011003400
(43) Veröffentlichungstag der Anmeldung: 11.12.2013
(73) Patentinhaber: Ate Antriebstechnik Und Entwicklungs GmbH, 88299 Leutkirch im Allgäu (DE)
(72) Erfinder: THALER, Wolfgang, 88299 Leutkirch-Herlazhofen (DE); VOHRER, Mark, 88299 Leutkirch (DE)
(74) Vertreter: Grünecker Patent- und Rechtsanwälte PartG mbB
(86) Internationale Anmeldenummer: PCT/EP2012/000397
(87) Internationale Veröffentlichungsnummer: WO 2012/104055

(56) Entgegenhaltungen:
- EP-A1- 1 104 086
- EP-A2- 0 854 558
- WO-A2-2010/106157
- CH-A5- 692 437
- DE-A1-102005 004 565
- DE-A1-102006 051 510

## Beschreibung

### Gebiet der Erfindung

Die Erfindung betrifft einen Elektromotor gemäß dem einleitenden Teil des Anspruches 1.

### Beschreibung des Standes der Technik

Elektromotoren der eingangs genannten Art werden beispielsweise in medizinischen Geräten, wie beispielsweise Bohrer, insbesondere Dentalbohrer, Fräsen, Knochenfräsen, Zahnfräsen und Sägen verwendet. Bei Anwendungen dieser Art sind jedoch verschiedene Besonderheiten zu beachten. U. a. ist eine kompakte Bauweise erforderlich, so dass das medizinische Instrument gut in der Hand liegt und ermüdungsfreies präzises Arbeiten ermöglicht. Der Motor sollte dabei leistungsfähig sein, so dass keine Drehzahländerungen unter Belastung auftreten. Weiterhin ist es bei medizinischen Instrumenten notwendig, den Arbeitsbereich mit zusätzlichen Medien zu versorgen. Beispielsweise muss der Arbeitsbereich zielgerichtet beleuchtet werden und mit Wasser und/oder Luft gekühlt oder gesäubert werden. Diese Medien müssen durch eigens dafür vorgesehene Versorgungsleitungen, die zwangsläufig durch den Motor hindurch oder an ihm vorbei geführt werden müssen, zum Arbeitsbereich (Operationsbereich) gebracht werden. Um die Medienversorgung zu gewährleisten, ist es entweder notwendig, den Durchmesser des Instruments zu vergrößern, wobei sich die Handhabung verschlechtert, oder der Motor muss verkleinert werden, wodurch sich das Laufverhalten und die Leistungsfähigkeit des Elektromotors verschlechtert.

In der Patentanmeldeschrift EP 2073347 A wird versucht, diesen Konflikt zwischen Baugröße und Motorleistungsfähigkeit zumindest teilweise zu umgehen, indem die Medienleitungen durch einen Rückschlusskörper des Stators, der den Motor verkapselt, durchgeführt werden. Da die Medienleitungen aber einen gewissen Durchmesser aufweisen müssen, beispielsweise 1 mm Innendurchmesser, muss zwangsläufig der Rückschlusskörper entsprechend dick ausgeführt werden. Damit muss der Rückschlusskörper dicker als eigentlich nötig ausgeführt werden. Weiterhin führen die dann auftretenden Inhomogenitäten im Rückschlusskörper durch die Leitungsdurchführungen zu einer Beeinflussung des Statormagnetfeldes, wodurch sich Laufeigenschaften verschlechtern können.

Ein anderer Ansatz wird in der Patentanmeldeschrift EP 0788779 A verfolgt. Die EP 0788779 beschreibt einen kollektorlosen Gleichstrommotor zum Antrieb eines ankuppelbaren dentalen Instruments, bei dem die Statorluftspaltwicklung Freiräume für die Medienleitungen aufweist. Die Freiräume werden erreicht, indem eine Vielzahl von dreieckförmigen Einzelspulen gleichmäßig und sich teilweise überlappend in Umfangsrichtung des Startors angeordnet werden, so dass sie einen geschlossenen Spulenring bilden. Durch diese Anordnung der Einzelspulen entsteht zum einen ein homogeneres Magnetfeld, bei dem Rastmomente des Rotors vermieden werden. Zum anderen bilden die überlappenden Einzelspulen einen geschlossenen Spulenring mit einem unebenen Außenumfang. Diese Unebenheiten entstehen durch die Überlappung und bilden Freiräume, durch die Medienleitungen geführt werden können. Dadurch kann der Rückschlusskörper symmetrisch und dünn ausgebildet werden und Unsymmetrien in der Magnetfeldführung werden vermieden. Dadurch können Rastmomente des Motors minimiert werden. Ein Nachteil dieser Anordnung ist jedoch, dass durch das homogenere Magnetfeld eine sensorlose Erfassung der Polstellung des magnetischen Rotors zur Drehzahlregelung des Motors insbesondere bei niedrigen Drehzahlen sehr schwierig wird. Weiterhin sind die Freiräume durch die überlappenden Spulen relativ klein, so dass der Volumenstrom durch die Medienleitung eingeschränkt ist. Ein weiterer Nachteil dieser Anordnung ist, dass die Spulenwicklungen nur als freitragende Stator Luftspaltwicklungen ausgeführt werden können. Das heißt, dass wegen der überlappenden Anordnung, die einzelnen Spulenwicklungen keinen weichmagnetischen Kern umschließen (freitragend) können. Dadurch verschlechtert sich der Wirkungsgrad des Motors.

Ein Elektromotor für ein dentales Handstück mit Durchführungen für Medienleitungen wird in der WO 2010/106157A2 offenbart. Ein Zahnmedizinisches Handstück mit Elektromotor mit Maßnahmen zum Schutz des Elektromotors bei Sterilisationsvorgängen ist in der DE 10 2006 051 510 A1 offenbart. Weiterer Stand der Technik ist CH 692 437.

Im Hinblick auf die Nachteile des Standes der Technik liegt der Erfindung die Aufgabe zu Grunde, einen kompakten Elektromotor mit hoher Leistungsfähigkeit bereitzustellen, der sehr präzise bis in niedrige Drehzahlbereiche exakt geregelt werden kann und der genügend Freiräume für Mediendurchführungen lässt.

### Zusammenfassung der Erfindung

Die Aufgabe wird gelöst durch einen erfindungsgemäßen Elektromotor mit einem drehbar gelagerten Rotormagnet und einen den Rotormagneten umgebenden Stator gemäß Anspruch 1. Der Elektromotor umfasst mindestens drei Spulenwicklungen und einen Wicklungsträger, wobei Spulenachsen der mindestens drei Spulenwicklungen radial zu einer Drehachse des Rotormagneten unter verschiedenen radialen Richtungen angeordnet sind. Die oben genannte Aufgabe wird insbesondere dadurch gelöst, dass die Spulenwicklungen so ausgeführt sind, dass sich zwischen mindestens zwei benachbarten Spulenwicklungen eine Lücke parallel zur Drehachse erstreckt, so dass mindestens eine sich in Längsrichtung erstreckende Medienleitung in die Lücke einbringen lässt. Im Gegensatz zur Spulenanordnung in der EP 0788 779 werden die Spulen der vorliegenden Erfindung nicht überlappend angeordnet, sondern so, dass eine Lücke zwischen benachbarten Spulen entsteht. Die Lücke kann durch entsprechende Auslegung der Wicklungen nach Bedarf variiert werden, je nach Platzbedarf für die Mediendurchführungen, ohne dass sich der Durchmesser des Elektromotors verändert. Weiterhin entstehen durch die Lücken zwischen benachbarten Spulen ausgeprägte Inhomogenitäten des magnetischen Flusses, die durch Erfassung der Gegeninduktionsspannungen in den Spulenwicklungen erfasst werden können. Die durch die Feldinhomogenitäten entstehenden Rastmomente können auf Grund dieser ausgeprägten Induktionssignale durch Gegensteuerung kompensiert werden, selbst bei sehr niedrigen Drehzahlen nahe 0 Umdrehungen pro Minute. Dies bedeutet, dass schon beim Anlaufen des Motors ein ausreichendes Signal zur Verfügung steht, mit dem die Stellung des Rotors erfasst werden kann, so dass eine Drehzahlregelung sowie ein glattes Bewegungsmoment in einem großen Umdrehungszahlbereich, beispielsweise 0 bis 200 000 Umdrehungen pro Minute möglich ist.

Beispielsweise kann ein kollektorloser Synchronmotor mit sensorloser Regelung verwendet werden. Hierzu wird eine Gegenspannung in die mindestens einer der mindestens drei Spulen zur Detektion der Rotorposition benutzt. Sensorlos bedeutet, dass keine separaten Sensoren zur Erfassung der Stellung des Rotors benötigt werden.

Der Wicklungsträger ist ein zylinderförmiger Hohlkörper, wobei eine Zylinderwand des zylinderförmigen Hohlkörpers Vorsprünge in radialer Richtung zur Zylinderachse entsprechend einer Anzahl der Spulenwicklungen aufweist, die von den Spulenwicklungen umgeben sind. Der Wicklungsträger enthält weichmagnetisches Material, um die Induktivität der Spulen zu erhöhen und die Effizienz des Motors zu verbessern. Die Vorsprünge dienen dabei als Befestigung- und Positioniereinrichtung der Spulenwicklungen. Gleichzeitig erhöhen die Vorsprünge die Spuleninduktivitäten und erhöhen die Kraftwirkung des erzeugten magnetischen Feldes auf den Rotor.

Die Vorsprünge können sich dabei vom Wicklungsträger radial nach außen oder nach innen erstrecken.

Erstrecken sich die Vorsprünge radial nach innen, ergibt sich eine verbesserte Kraftwirkung der Magnetfelder der einzelnen Spulenwicklungen auf den Rotor, da die Spulenwicklungen näher am Rotor angeordnet sind.

Wenn sich die Vorsprünge radial nach außen erstrecken, vereinfacht sich die Fertigung des Stators, da die Spulenwicklungen von außen auf die Vorsprünge des Wicklungsträgers aufgesetzt werden können.

In einer Ausführungsform ist der Wicklungsträger aus einem Stapel gestanzter Bleche hergestellt. Die Realisierung des Wicklungsträgers mit einem Stapel gestanzter Bleche hat den Vorteil, dass die komplexe Form des Wicklungsträgers mit den Vorsprüngen mit einem einfachen technischen Verfahren realisiert werden kann. Weiterhin werden durch die Verwendung eines Stapels gestanzter Bleche Wirbelströme im Wicklungsträger reduziert insbesondere wenn die gestanzten Bleche voneinander isoliert sind. In einer besonderen Ausführungsform bestehen die gestanzten Bleche aus einem Nickelstahl. Nickelstahl hat zum einen eine gute feromagnetische (weichmagnetische) Eigenschaft, und ist zum anderen besonders korrosionsbeständig. Insbesondere wenn erfindungsgemäß Elektromotoren in medizinischen Instrumenten verwendet wird, wie z. B. Dentalbohrer, Knochenfräsen, etc., muss der Elektromotor gegenüber korrosiven Umgebungen beständig sein, da medizinische Instrumente regelmäßig Sterilisationsprozessen mit aggressiven Chemikalien ausgesetzt sind.

Alternativ kann der Wicklungsträger auch als ein Kunststoffspritzgussformteil mit weichmagnetischen Materialeinschlüssen, wie beispielsweise Eisenpulver, gefertigt sein. Insbesondere bei hohen Stückzahlen können dadurch Fertigungskosten reduziert werden. Darüber hinaus können bei entsprechender Ausgestaltung der Gussform Kanten an den Vorsprüngen des Wicklungsträgers vermieden werden, so dass eine Verletzung der Isolierung der Wicklungsdrähte vermieden werden kann. Weiterhin sind die verwendbaren Kunststoffmaterialien Nichtleiter, so dass die Entstehung von Wirbelströmen minimiert wird. Zu beachten ist jedoch, dass der Kunststoff eine ausreichende Spannungsfestigkeit aufweist, da die besonders bei hohen Drehzahlen auftretenden großen Induktionsspannungen einen Durchschlag im Kunststoff bewirken könnten.

In einer weiteren Alternative ist der Wicklungsträger aus einem gesinterten Keramikformteil mit weichmagnetischen Materialeinschlüssen, wie z. B. Eisenpulver, gefertigt. Wicklungsträger aus keramischen Werkstoffen weisen eine hohe mechanische Festigkeit, eine hohe elektrische Spannungsfestigkeit und eine hohe Widerstandsfähigkeit gegenüber korrosiven Materialien, wie sie z. B. in Sterilisationsprozessen verwendet werden, auf.

In einer besonderen Ausführungsform ist der Rotormagnet ein hermetisch dicht ummantelter Permanentmagnet, der rotationssymmetrisch an einer Achswelle befestigt ist. Durch den symmetrischen Aufbau können hohe Drehzahlen erreicht werden ohne die Lager auf Grund von hoher Unwucht stark zu belasten. Weiterhin dient die hermetisch dichte Ummantelung dazu, den Permanentmagneten vor Korrosion zu schützen. Da Permanentmagnete aus hartmagnetischen Materialien gefertigt sind, die sehr korrosionsanfällig sind, müssen insbesondere bei der Anwendung in medizinischen Instrumenten korrosionsanfällige Komponenten geschützt werden. Insbesondere muss die Permanentmagnetummantelung aus einem Material bestehen, das nicht quillt und nicht korrodiert unter dem Einfluss von Sterilisationsprozessen. Da Sterilisationsmaterialien oft die Wirkung haben, dass Kunststoffvergussmaterialien quellen und dass der Permanentmagnet schnell korrodiert, kann ein ungeschützter oder unzureichend geschützter Permanentmagnet nach Sterilisationsprozessen seine Laufeigenschaften schnell verlieren und möglicherweise blockieren. Beispielsweise kann die Permanentmagnetummantelung aus einer nicht magnetischen Hülse aus Stahl, aus Kunststoff, wie z. B. Teflon oder ein kohlenstofffaserverstärkter Kunststoff sein. Alternativ dazu kann der Rotormagnet ein Spritzgussformteil oder ein gesintertes Formteil mit darin eingeschlossenen magnetischen Partikeln, beispielsweise magnetisiertes Eisenpulver, sein.

Vorzugsweise sind die vorher genannten Ausführungsformen des Elektromotors besonders geeignet zur Verwendung als chirurgisches Instrument oder als zahnmedizinisches Instrument, insbesondere zur Verwendung als Bohrer, Dentalbohrer, Knochenfräse oder Knochensäge.

### Kurze Beschreibung der Figuren

Die Erfindung wird nachfolgend an Hand von Beispielen unter Bezugnahme auf die begleitenden Zeichnungen näher beschrieben, bei denen
Fig. 1 einen Querschnitt eines Elektromotors zeigt,
Fig. 2 einen anderen Querschnitt des Elektromotors zeigt,
Fig. 3 eine erfindungsgemäße Variante des Beispiels gemäß der Fig. 1 zeigt; und
Fig. 4 eine andere erfindungsgemäße Variante des Beispiels gemäß Fig. 1 zeigt.

### Ausführliche Beschreibung von Ausführungsformen

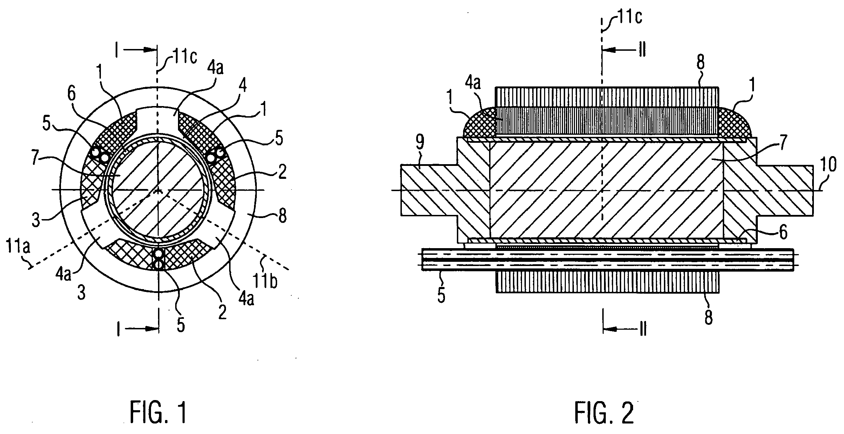

Die Fig. 1 und 2 zeigen Querschnitte eines Elektromotors. Fig. 1 zeigt einen Schnitt quer zur Rotationsachse des Rotors und Fig. 2 zeigt einen Querschnitt längs zur Rotationsachse des Rotors.

In Fig. 1 bezeichnen die Bezugszeichen 1, 2 und 3 Spulenwicklungen, das Bezugszeichen 4 bezeichnet den Wicklungsträger, das Bezugszeichen 4a bezeichnet einen Vorsprung des Wicklungsträgers 4, das Bezugszeichen 5 bezeichnet Medienleitungen, das Bezugszeichen 6 bezeichnet eine hermetische Ummantelung eines Rotormagneten 7, und das Bezugszeichen 8 bezeichnet einen Rückschlusskörper. Die Bezugszeichen 11a, 11b und 11c bezeichnen Achsen der Spulenwicklungen 1, 2 und 3. Die Spulenwicklungen 1, 2 und 3 umgeben die Vorsprünge 4a des Wicklungsträgers 4.

Bei Bestromung der Spulenwicklungen 1, 2 und 3 entsteht ein Magnetfeld in den Vorsprüngen 4a parallel zu den Spulenachsen 11a, 11b und 11c, so dass ein Drehmoment auf den im Innern des Wicklungsträgers 4 drehbar gelagerten Rotormagneten 7 ausgeübt werden kann.

Die Spulenwicklungen 1, 2 und 3 sind so angefertigt, dass eine Lücke zwischen benachbarten Spulenwicklungen besteht. In dieser Lücke können Medienleitungen 5 eingebettet werden, durch die Wasser, Luft und Licht geführt werden können. Der Rückschlusskörper 8 verkapselt den Elektromotor nach außen. Der Wicklungsträger 4, die Spulen 1, 2 und 3 und der Rückschlusskörper 8 bilden den Stator des Elektromagneten. Der Wicklungsträger 4 und der Rückschlusskörper 8 beinhalten weichmagnetische Materialien, um die Induktivitäten der Spulenwicklungen 1, 2 und 3 zu erhöhen, so dass der Wirkungsgrad des Elektromotors verbessert wird. Der Wicklungsträger 4 ist als zylinderförmiger Hohlkörper ausgebildet. Insbesondere ist der Hohlraum ein kreisförmiger Zylinder, in dem der zylinderförmige, den Rotormagneten 7 enthaltende, Rotor drehbar eingepasst ist. Der Innendurchmesser des zylinderförmigen Hohlkörpers ist etwas größer als der Außendurchmesser des zylinderförmigen Rotors, so dass sich der Rotor relativ zum Stator drehen kann. Der zylinderförmige Rotormagnet 7 ist hermetisch dicht mit einer Ummantelung 6 versehen, die das leicht korrodierende Permanentmagnetmaterial vor Korrosion schützt.

Im Betrieb wird beim Anfahren des Motors mindestens eine der mindestens drei Spulenwicklungen bestromt. Aus der Induktionsspannung mindestens einer anderen der mindestens drei Spulenwicklungen ergibt sich die Lage der Pole des Rotormagneten 7, so dass die Stellung des Permanentmagneten (Rotormagneten 7) relativ zu den Spulenwicklungen 1, 2 und 3 bestimmt werden kann. Danach werden die Spulenwicklungen 1, 2 und 3 so bestromt, dass ein maximales Anlaufdrehmoment entsteht. Die Induktionsspannungen in den Spulenwicklungen, die durch den sich drehenden Rotormagneten 7 entstehen, werden ständig überwacht und die Bestromung der Spulenwicklungen 1, 2 und 3 wird permanent angepasst, bis die Solldrehzahl erreicht ist. Bei Belastung des Elektromotors, bei der eine Drehzahländerung auftritt, wird die Drehzahländerung durch die Überwachung der Gegeninduktion registriert und die Bestromung so angepasst, dass das Drehmoment ausreicht, die Nenndrehzahl zu erreichen. Durch die ausgeprägte Unsymmetrie der Magnetfelder der drei Spulenwicklungen können bereits sehr kleine Bewegungen des Rotormagneten 7 erfasst werden und eine Regelung von sehr kleinen Drehzahlen von beinahe Null Umdrehungen pro Minute bis zu sehr hohen Drehzahlen, beispielsweise von ca. 200 000 Umdrehungen pro Minute, können realisiert werden. Um Rastmomente besser zu kompensieren, ist es möglich, den Motor mit einer Vektorregelung zu betreiben. Dafür werden alle Spulenwicklungen 1, 2 und 3 gleichzeitig bestromt. Je nach Last und Drehzahl werden die Phasen und die Spannungen an den drei Spulenwicklungen unabhängig voneinander aktiv der Rotorlage entsprechend geregelt.

Fig. 2 zeigt einen Schnitt entlang der Linie I-I aus Fig. 1. In Fig. 2 sind die Medienleitungen 5 nun im Längschnitt gezeigt. Der Stator wird durch den Rückschlusskörper 8, den Wicklungsträger 4 mit den Vorsprüngen 4a und den Spulenwicklungen 1, 2 und 3 gebildet. In Fig. 2 ist nur die Spulenwicklung 1 zu sehen. Der Rotor wird gebildet aus dem Rotormagneten 7, der Rotorachse 9 und der Permanentmagnetummantelung 6 des Rotormagneten 7. Der Rotor ist als kreisförmiger Zylinder ausgebildet, wobei der Kreismittelpunkt auf der Drehachse 10 des Rotors liegt, so dass sich der Rotor um die Drehachse 10 relativ zur Stellung des Stators drehen kann. Bei einem Schnitt entlang der Schnittachse II-II gelangt man wieder zur Darstellung gemäß der Fig. 1.

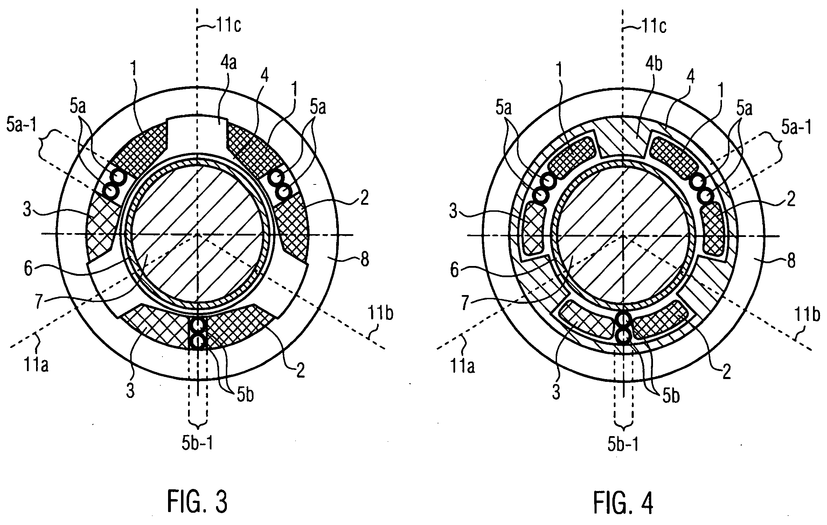

Obwohl die in den Figuren 1 und 2 sowie in den folgenden Figuren 3 und 4 gezeigten Beispiele drei Spulen aufweisen, ist die vorliegende Erfindung nicht auf drei Spulen beschränkt, sondern es können auch mehr als drei Spulen angeordnet werden.

In dem Beispiel gemäß den Figuren 1 und 2 sind jeweils 2 Medienleitungen in radialer Richtung hintereinander in jeweils einer Lücke zwischen zwei benachbarten Spulenwicklungen (beispielsweise zwischen Spulenwicklung 1 und Spulenwicklung 2) angeordnet. Um die Verwendbarkeit als Dentalbohrer zu gewährleisten, sollte der Außendurchmesser des Rückschlusskörpers 8 nicht größer als 2 cm vorzugsweise 1,5 cm bis 1,85 cm sein. Die Medienleitungen 5 können dann in der in Fig. 1 und 2 gezeigten Anordnung einen Außendurchmesser von jeweils 1 mm aufweisen. Der Wicklungsträger 4 und der Rückschlusskörper 8 sind aus einem weichmagnetischen Material oder einem Material mit weichmagnetischen Komponenten gefertigt. Bei der Verwendung als medizinisches Instrument ist insbesondere darauf zu achten, dass die verwendeten Materialien sterilisierbar sind, d. h. die verwendeten Materialien dürfen beim Sterilisationsprozess ihre Form nicht verändern, beispielsweise durch Quellen und dürfen auch ihre chemische Zusammensetzung, beispielsweise durch Oxidieren nicht verändern. In fertigungstechnischer Hinsicht ist die Verwendung eines Stapels von gestanzten Nickelstahlblechen für den Wicklungsträger 4 vorteilhaft. Nickelstahl ist ein korrosionsbeständiges feromagnetisches (weichmagnetisches) korrosionsbeständiges Material. Weiterhin lässt sich die komplizierte Geometrie des Wicklungsträgers 4 durch Stanzen leicht erreichen. Eine Ausführung als Stapel gestanzter Blech ermöglicht auch, Wirbelstromverluste zu minimieren.

Bei höheren Stückzahlen kann aus einem fertigungstechnischen Gesichtspunkt eine Ausführung als spritzgussgefertigtes Formteil mit weichmagnetischen Einschlüssen oder als gesintertes Keramikformteil mit weichmagnetischen Einschlüssen vorteilhaft sein. Bei Verwendung von Kunststoffen für spritzgussgefertigte Teile ist darauf zu achten, dass der Kunststoff eine ausreichende Spannungsfestigkeit aufweist. Als weichmagnetische Einschlüsse kann zum Beispiel Eisenpulver verwendet werden. Als Rückschlusskörper 8 kann eine Hülse verwendet werden, die über den Wicklungsträger mit den Spulenwicklungen gesteckt wird. Vorzugsweise besteht der Rückschlusskörper 8 aus einem weichmagnetischen Material oder einem Material mit weichmagnetischen Einschlüssen. Beispielsweise kann eine Hülse aus Nickelstahl, eine spritzgussgefertigte Hülse mit weichmagnetischen Einschlüssen, wie Eisenpulver oder eine gesinterte Keramikhülse mit weichmagnetischen Einschlüssen verwendet werden.

Fig. 3 zeigt eine erfindungsgemäße Ausführungsform des Elektromotors mit einer anderen Anordnung der Medienleitungen. Das Bezugszeichen 5b zeigt eine Anordnung, bei der zwei Medienleitungen in radialer Richtung hintereinander angeordnet sind. Eine solche Anordnung wird auch in Fig. 1 und Fig. 2 gezeigt. Eine Anordnung hintereinander in radialer Richtung erfordert eine Lücke 5b-1 zwischen den benachbarten Spulenwicklungen 2 und 3. Das Bezugszeichen 5a zeigt eine Anordnung von zwei Medienleitungen, die in einer Umfangsrichtung nebeneinander angeordnet sind. In diesem Fall ist ein größerer Durchmesser der Medienleitungen 5a möglich, ohne dass der gesamte Außendurchmesser von ca. 15 bis 20 mm vergrößert werden muss. Beispielsweise kann der Außendurchmesser der Medienleitungen 5a jeweils 1,5 bis 2 mm im Vergleich zu den hintereinander angeordneten Medienleitungen 5b mit einem Außendurchmesser von jeweils 1 mm betragen. Dabei benötigen die in zirkularer Richtung nebeneinander liegenden Medienleitungen 5a eine größere Lücke 5a-1 zwischen den benachbarten Spulenwicklungen 1 und 3. Die in Fig. 3 gezeigte Darstellung weist drei Spulenwicklungen 1, 2 und 3 mit entsprechenden drei Lücken 5b-1 bzw. 5a-1 auf, wobei eine schmale Lücke 5b-1 und zwei breite Lücken 5a-1 realisiert werden. Dies führt zu einer weiteren Unsymmetrie des Magnetfeldes, wodurch eine sensorlose Detektion der Stellung des Magnetrotors 7 bei noch geringeren Drehzahlen möglich wird.

Obwohl in der Fig. 3 eine unsymmetrische Anordnung der Medienleitungen mit zwei großen Lücken 5a-1 und einer kleinen Lücke 5b-1 gezeigt ist, ist eine andere unsymmetrische Anordnung mit zwei kleinem Lücken 5b-1 und einer großen Lücke 5a-1 möglich. Weiterhin ist auch eine symmetrische Anordnung mit drei breiten Lücken 5a-1 möglich, beispielsweise wenn Medienleitungen mit erhöhtem Durchmesser erforderlich sind.

Die Bezugszeichen in Fig. 3, die identisch zu dem Bezugszeichen in Fig. 1 sind, bezeichnen dabei jeweils die gleichen technischen Merkmale und zur Erläuterung der im Zusammenhang mit Fig. 3 nicht benannten Elemente wird auf Fig. 1 verwiesen.

Fig. 4 zeigt eine alternative erfindungsgemäße Ausführungsform des in Fig. 1 gezeigten Elektromotors. In Fig. 4 ragen die Vorsprünge 4b des Wicklungsträgers 4 radial nach innen, so dass die Spulenwicklungen 1, 2 und 3 auf der Innenseite des als zylinderförmigen Hohlkörpers ausgebildeten Wicklungsträgers 4 auf den Vorsprüngen 4b sitzen. Diese Bauform hat den Vorteil, dass die magnetische Kraftübertragung von den Vorsprüngen 4b auf den Magnetrotor 7 direkter erfolgen kann und damit die Kraftübertragung effizienter ist. In Fig. 4 ist wieder eine unsymmetrische Anordnung der Medienleitungen 5a und 5b wie in Fig. 3 gezeigt. Wie in Verbindung mit Fig. 3 beschrieben, ist auch hier jede mögliche Kombination von radialer oder zirkularer Anordnung der Medienleitungen 5a bzw. 5b möglich. Wie in Fig. 3 wird auch in Fig. 4 bei den hier nicht erwähnten Bezugszeichen auf die Fig. 1 verwiesen.

Ein solchermaßen gestalteter Elektromotor, wie er beispielhaft mit Bezug auf die Figuren 1 bis 4 beschrieben wurde, kann vorteilhaft für medizinische Instrumente, insbesondere Bohrer, Dentalbohrer oder Knochenfräsen verwendet werden, da der Elektromotor sterilisierbar und für hohe Drehzahlbereiche, beispielsweise 0-20.000 Umdrehungen/Minute, 0-60.000 Umdrehungen/Minute und sogar 0-200.000 Umdrehungen/Minute ausgeführt werden kann. Weiterhin kann der Elektromotor sehr belastbar, also Drehzahlstabil unter Last ausgelegt werden.

Obwohl die vorliegenden Offenbarung in Bezug auf Ausführungsformen beschrieben wird, wie sie in der vorangegangen Beschreibung veranschaulicht werden, ist die ausführliche Beschreibung nicht dafür vorgesehen, die vorliegende Offenbarung auf bestimmte Ausführungsformen einzuschränken, sondern die beschriebenen Ausführungsformen sollen nur die verschiedenen Aspekte der vorliegenden Erfindung beispielhaft erläutern, deren Umfang durch die beiliegenden Ansprüche definiert ist.

## Patentansprüche

1. Elektromotor mit einem drehbar gelagerten Rotormagnet (7) und einem den Rotormagneten (7) umgebenden Stator, der mindestens drei Spulenwicklungen (1, 2, 3) und einen Wicklungsträger (4) umfasst, wobei Spulenachsen (11a, 11 b, 11 c) der mindestens drei Spulenwicklungen (1, 2, 3) radial zu einer Drehachse (10) des Rotormagneten (7) unter verschiedenen radialen Richtungen angeordnet sind;
die Spulenwicklungen (1, 2, 3) so ausgeführt sind, dass sich zwischen wenigstens zwei benachbarten Spulenwicklungen eine Lücke (5a-1, 5b-1) parallel zur Drehachse (10) erstreckt, in der mindestens eine sich in Längsrichtung erstreckende Medienleitung (5, 5a, 5b) vorgesehen ist,
**dadurch gekennzeichnet,**
**dass** der Wicklungsträger (4) aus einem weichmagnetischen Material besteht, und
**dass** der Wicklungsträger (4) ein zylinderförmiger Hohlkörper ist, wobei eine Zylinderwand des zylinderförmigen Hohlkörpers Vorsprünge (4a, 4b) in radialer Richtung zur Zylinderachse entsprechend einer Anzahl der Spulenwicklungen (1, 2, 3) aufweist, wobei jeder der Vorsprünge (4a, 4b) von jeweils einer der Spulenwicklungen (1, 2, 3) umgeben ist,
wobei eine lichte Weite der Lücke (5a-1, 5b-1) so ausgelegt ist, dass mindestens zwei Medienleitungen (5a, 5b) durchgeführt werden können,
wobei jeweils zwei Medienleitungen in einer Lücke in Umfangrichtung nebeneinander angeordnet sind und jeweils zwei Medienleitungen in einer anderen Lücke in Radialrichtung nebeneinander angeordnet sind.

2. Elektromotor nach Anspruch 1, wobei der Elektromotor ein kollektorloser Synchronmotor ist.

3. Elektromotor nach Anspruch 1 oder 2, worin die Vorsprünge (4a) radial nach außen zeigen.

4. Elektromotor nach Anspruch 1 oder 2, worin die Vorsprünge (4b) radial nach innen zeigen.

5. Elektromotor nach einem der vorangehenden Ansprüche, worin der Wicklungsträger (4) aus einem Stapel gestanzter Bleche besteht.

6. Elektromotor nach einem der Ansprüche 1 - 5, worin die gestanzten Bleche aus einem Nickelstahl bestehen.

7. Elektromotor nach einem der Ansprüche 1 - 5, worin der Wicklungsträger (4) ein Kunststoffspritzgussformteil mit weichmagnetischen Materialeinschlüssen oder ein gesintertes Keramikformteil mit weichmagnetischen Materialeinschlüssen ist.

8. Elektromotor nach einem der vorangehenden Ansprüche, worin der Rotormagnet (7) ein hermetisch dicht ummantelter Permanentmagnet ist, der rotationssymmetrisch an einer Achswelle (9) befestigt ist.

9. Elektromotor nach Anspruch 8, worin eine Permanentmagnetummantelung (6) aus einem Material besteht, das nicht quillt und nicht korrodiert unter dem Einfluss von Sterilisationsprozessen.

10. Elektromotor nach Anspruch 9, worin die Permanentmagnetummantelung (6) eine nichtmagnetische Hülse aus Stahl, Kunststoff oder kohlenstofffaserverstärkter Kunststoff ist.

11. Elektromotor nach einem der vorangehenden Ansprüche, worin der Rotormagnet (6) ein Spritzgussformteil oder ein gesintertes Formteil mit darin eingeschlossenen magnetischen Partikeln ist.

12. Elektromotor nach Anspruch 11, worin die magnetischen Partikel aus einem Eisen Pulver gebildet werden.

13. Elektromotor nach einem der vorangehenden Ansprüche, der zur Verwendung als chirurgisches Instrument oder als zahnmedizinisches Instrument ausgelegt ist, insbesondere zur Verwendung als Bohrer, Dentalbohrer, Knochenfräse oder Knochensäge.

## Claims

1. Electric motor with a rotatably mounted rotor magnet (7) and a stator enclosing the rotor magnet (7), said stator comprising at least three coil windings (1,2, 3) and a winding carrier (4), wherein coil axes (11 a, 11 b, 11 c) of the at least three coil windings are disposed radially to an axis of rotation (10) of the rotor magnet (7) in various radial directions;
the coil windings (1,2, 3) are designed such that a gap (5a-1, 5b-1) that is parallel to the axis of rotation (10) extends between at least two adjacent coil windings, in which at least one media line (5, 5a, 5b) extends in a longitudinal direction,
**characterized in that**
the winding carrier (4) consists of a soft-magnetic material, and
the winding carrier (4) is a cylindrical hollow body, wherein a cylinder wall of the cylindrical hollow body comprises projections (4a, 4b) in the radial direction to the cylinder axis corresponding to a number of coil windings (1,2, 3) wherein each of the projections (4a, 4b) is surrounded by one of the coil windings (1,2, 3),
wherein the clearance of the gap (5a-1, 5b-1) is adapted to receive at least two media lines (5, 5a, 5b), and
respective two media lines in one gap are arranged side by side in a circumferential direction with regard to the axis of rotation, and respective two media lines in another gap are arranged side by side in a radial direction to the axis of rotation.

2. Electric motor according to claim 1, wherein the electric motor is a collectorless synchronous motor.

3. Electric motor according to claim 1 or 2, wherein the projections (4a) radially face outwards.

4. Electric motor according to claim 1 or 2, wherein the projections (4b) radially face inwards.

5. Electric motor according to any one of the preceding claims, wherein the winding carrier (4) consists of a stack of stampings.

6. Electric motor according to claim 5, wherein the stampings consist of a nickel steel.

7. Electric motor according to any one of the claims 1 - 5, wherein the winding carrier (4) is a molded plastic part with soft-magnetic material inclusions, or a sintered ceramic part with soft-magnetic material inclusions.

8. Electric motor according to any one of the preceding claims, wherein the rotor magnet is a hermetically tightly enclosed permanent magnet which is rotationally symmetrically fixed to an axle shaft.

9. Electric motor according to claim 8, wherein a permanent magnet casing (6) consists of a material which does not swell and does not corrode under the influence of sterilization processes.

10. Electric motor according to claim 9, wherein the permanent magnet casing (6) is a non-magnetic sleeve of steel, plastic or carbon reinforced plastic.

11. Electric motor according to any one of the preceding claims, wherein the rotor magnet (6) is an injection-molded part or a sintered part with magnetic particles included therein.

12. Electric motor according to claim 11, wherein the magnetic particles are formed of an iron powder.

13. Electric motor according to any one of the preceding claims, which is designed to be used as a surgical instrument or as a dental instrument, in particular to be used as a drill, a dental drill, a bone cutter or a bone saw.

## Revendications

1. Moteur électrique comportant un aimant de rotor (7) monté rotatif et un stator qui entoure l'aimant de rotor (7) et comprend au moins trois enroulements de bobinage (1, 2, 3) et un support d'enroulements (4), moteur électrique
dans lequel des axes de bobinage (11a, 11b, 11c) desdits au moins trois enroulements de bobinage (1, 2, 3) sont agencés radialement par rapport à un axe de rotation (10) de l'aimant de rotor (7), sous des directions radiales différentes,
dans lequel les enroulements de bobinage (1, 2, 3) sont réalisés de manière telle, qu'entre au moins deux enroulements de bobinage voisins s'étende, parallèlement à l'axe de rotation (10), un interstice (5a-1, 5b-1) dans lequel est prévue au moins une conduite de fluide (5, 5a, 5b) s'étendant dans la direction longitudinale, **caractérisé**
**en ce que** le support d'enroulements (4) est constitué d'un matériau magnétique doux, et
**en ce que** le support d'enroulements (4) est un corps creux de forme cylindrique, une paroi cylindrique du corps creux de forme cylindrique présentant des proéminences (4a, 4b) dans une direction radiale à l'axe de cylindre, qui sont d'un nombre correspondant à celui des enroulements de bobinage (1, 2, 3), chacune des proéminences (4a, 4b) étant entourée respectivement par l'un des enroulements de bobinage (1, 2, 3),
une largeur libre de l'interstice (5a-1, 5b-1) étant dimensionnée de manière à pouvoir y faire passer au moins deux conduites de fluide (5a, 5b),
deux conduites de fluide dans un interstice étant respectivement agencées côte à côte dans la direction périphérique, et deux conduites de fluide dans un autre interstice étant respectivement agencées côte à côte dans la direction radiale.

2. Moteur électrique selon la revendication 1, d'après lequel le moteur électrique est un moteur synchrone sans collecteur.

3. Moteur électrique selon la revendication 1 ou la revendication 2, dans lequel les proéminences (4a) sont dirigées radialement vers l'extérieur.

4. Moteur électrique selon la revendication 1 ou la revendication 2, dans lequel les proéminences (4b) sont dirigées radialement vers l'intérieur.

5. Moteur électrique selon l'une des revendications précédentes, dans lequel le support d'enroulements (4) est constitué d'un empilement de tôles découpées.

6. Moteur électrique selon l'une des revendications 1 - 5, dans lequel les tôles découpées sont réalisées en un acier au nickel.

7. Moteur électrique selon l'une des revendications 1 - 5, dans lequel le support d'enroulements (4) est une pièce de moulage en matière plastique moulée par injection avec des inclusions de matériau magnétique doux, ou une pièce de moulage en céramique frittée avec des inclusions de matériau magnétique doux.

8. Moteur électrique selon l'une des revendications précédentes, dans lequel l'aimant de rotor (7) est un aimant permanent enveloppé de manière hermétiquement étanche, qui est fixé selon une symétrie de rotation sur un arbre d'axe (9).

9. Moteur électrique selon la revendication 8, dans lequel une enveloppe d'aimant permanent (6) est constituée d'un matériau qui ne gonfle pas et ne se corrode pas sous l'effet de processus de stérilisation.

10. Moteur électrique selon la revendication 9, dans lequel l'enveloppe d'aimant permanent (6) est un fourreau non magnétique en acier, matière plastique ou matière plastique renforcée de fibres de carbone.

11. Moteur électrique selon l'une des revendications précédentes, dans lequel l'aimant de rotor (7) est une pièce de moulage moulée par injection ou une pièce de moulage frittée dans laquelle sont incluses des particules magnétiques.

12. Moteur électrique selon la revendication 11, dans lequel les particules magnétiques sont formées par une poudre de fer.

13. Moteur électrique selon l'une des revendications précédentes, qui est conçu pour être utilisé en tant qu'instrument chirurgical ou instrument médical dentaire, notamment pour être utilisé en tant que perceuse, perceuse-fraiseuse dentaire, fraiseuse à os ou scie à os.
